(19) Europäisches Patentamt
European Patent Office
Office européen des brevets

(11) Publication number: **0 218 010**
**A2**

# (12) EUROPEAN PATENT APPLICATION

(21) Application number: 86108092.7

(51) Int. Cl.4: G 01 N 33/533, C 09 B 11/08

(22) Date of filing: 13.06.86

(30) Priority: 10.07.85 US 753476

(43) Date of publication of application: 15.04.87 Bulletin 87/16

(84) Designated Contracting States: BE DE FR IT

(71) Applicant: ABBOTT LABORATORIES, 14th Street and Sheridan Road North St, North Chicago Illinois 60064 (US)

(72) Inventor: Heiman, Daniel F., 407 Drake, Libertyville Illinois 60048 (US)
Inventor: Cantarero, Luis A., 1319 Dunleer, Mundelein Illinois 60060 (US)
Inventor: Groth, Marc C., 25 Monica Drive, Lake Villa Illinois 60046 (US)
Inventor: Wang, Philip P., 608 Dawes Street, Libertyville Illinois 60048 (US)

(74) Representative: Modiano, Guido et al, MODIANO, JOSIF, PISANTY & STAUB Modiano & Associati Via Meravigli, 16, I-20123 Milan (IT)

## (54) Ligand detection method and substituted carboxyfluorescein tracers therefor.

(57) This disclosure relates to a method and reagents for determining ligands in biological fluids such as serum, plasma, spinal fluid, amnionic fluid and urine. This disclosure also relates to a novel class of tracer compounds employed as reagents in fluorescence polarization immunoassays.

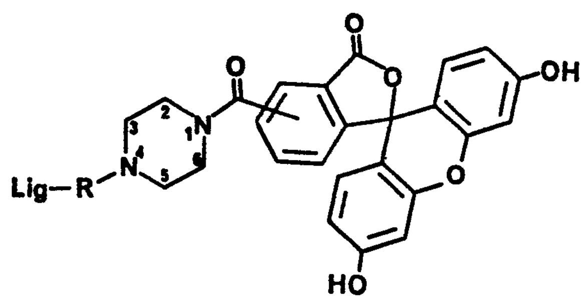

EP 0 218 010 A2

# SUBSTITUTED CARBOXYFLUORESCEINS

## BACKGROUND OF THE INVENTION

### Technical Field

The present invention relates to a method and reagents for determining ligands in biological fluids such as serum, plasma, spinal fluid, amnionic fluid and urine, and to the methods for making the reagents. The present invention also relates to a novel class of fluorescein derivatives which can be used as reagents in fluorescent polarization immunoassays.

### Background Art

Competitive binding immunoassays for measuring ligands are based on the competition between a ligand in a test sample and a labeled reagent, referred to as a tracer, for a limited number of receptor binding sites on antibodies specific to the ligand and tracer. The concentration of ligand in the sample determines the amount of tracer that will specifically bind to an antibody. The amount of tracer-antibody complex produced can be quantitatively measured and is inversely proportional to the quantity of ligand in the test sample.

Fluorescence polarization immunoassay techniques are based on the principle that a fluorescent labeled compound, when excited by plane polarized light, will emit fluorescence having a degree of polarization inversely related to its rate of rotation. Specifically, when a molecule such as a tracer-antibody complex having a fluorescent label is excited with plane polarized light, the emitted light remains highly polarized because the fluorophore is constrained from rotating between the time when light is absorbed and when it is emitted. When a "free" tracer compound (i.e., unbound to an antibody) is excited by plane polarized light, its rotation is much faster than that of the corresponding tracer-antibody complex; therefore, the emitted light is depolarized to a much greater extent. Thus, fluorescence polarization provides a quantitative means for measuring the amount of tracer-antibody complex produced in a competitive binding immunoassay.

Fluorescence polarization techniques have been applied in U.S. Patent No. 4,420,568 to Wang, et al., commonly assigned herewith, which is directed to the use of a triazinylamino-fluorescein moieties as fluorophores. Various other fluorescent labeled compounds are known in the art. For example, U.S. Patent No. 3,998,943 describes the preparation of a fluorescently labeled insulin derivative using fluorescein isothiocyanate (FITC) as the fluorescent label and a fluorescently-labeled morphine derivative using 4-aminofluorescein hydrochloride as the fluorescent label. Carboxyfluorescein has also been used for analytical determinations. R. C. Chen, Analytical Letters, 10, 787 (1977) describes the use of carboxyfluorescein to indicate the activity of phospholipase. The carboxyfluorescein is described as

encapsulated in lecithin liposomes, and as fluorescing only when released by the hydrolysis of lecithin. U.S. Patent Application Serial No. 329,974, filed December 11, 1981, and commonly assigned herewith, discloses a class of carboxyfuorescein derivatives which are directly bonded to a ligand-analog. U.S. Patent No.4,476,229 to Fino, et al., and also commonly assigned herewith describes a series of amino acid amido derivatives of carboxyfluorescein useful as reagents in fluorescence polarization immunoassays.

SUMMARY OF THE INVENTION

The present invention offers an advance in the art beyond the foregoing, in that the cyclic piperazino moiety herein employed provides a semirigid and non-rotating spacer and linker between the ligand analog and the fluorescent label. Tracers prepared by direct coupling of carboxyfluoresceins or aminofluoresceins to the ligand analog frequently exhibit weak binding to antibody or receptor because the bulk of the fluorescein molecule prevents proper entry into the binding site. Tracers in which fluorescein is coupled to the ligand analog through a linear linking group may exhibit low polarization even when bound because there is little resistance to rotation and bending in such a linking arm.

The present invention encompasses a method for determining ligands in a sample comprising:

a) intermixing with the sample a biologically acceptable salt of a tracer represented by the structural formula shown in Figure 1 of the drawings, wherein

Lig is a ligand analog;

R is an additional linking group consisting of from zero to 12 carbon, sulfur, nitrogen or oxygen

atoms, together with sufficient hydrogens to satisfy valences of the above-named atoms which are not otherwise satisfied, and bearing a reactive functional group which has been attached to the 4-position amine nitrogen of the piperazine ring; and wherein the ligand analog has a maximum of one common epitope with the ligand so as to be specifically recognizable by a common antibody or other receptor binding site and an antibody capable of specifically recognizing said ligand and said tracer, whereby a tracer-antibody complex is formed; and

b) determining the amount of tracer-antibody complex formed in step a) by fluorescence polarization technique, as a measure of the concentration of the ligand in the sample.

The invention further encompasses certain novel tracers and biologically acceptable salts thereof, which are useful as reagents in the above-described method.

## BRIEF DESCRIPTION OF THE DRAWINGS

Figure 1 shows a general structural formula for the tracers of the present invention together with the numbering system for the piperazine ring. The use of a bond not attached to a particular carbon atom of the benzoic acid ring is meant to signify that the carbonyl group may be bonded to any of the carbon atoms in this ring which are not attached to the carboxylate group or the xanthene ring system.

Figure 2 shows the alternate structural formulae and names for the fluorescein moieties included in the tracers of the present invention.

Figures 3 and 4 show structural formulae for preferred fluorescein isomers of the tracers of the present invention.

Figures 5 and 6 show strucutural formulae for two isomers of carboxyfluorescein which are used as starting materials in the synthesis of preferred tracers of the present invention.

Figures 7 and 8 show structural formulae for two isomers of intermediates which can serve as precursors for the preferred tracers of Figures 3 and 4.

Figure 9 shows the various linkages which may couple the ligand analog to the piperazinocarboxyfluorescein moiety in the tracers of the present invention. In this Figure, the symbol "Fl" represents a fluorescein moiety; $R_1$ represents an alkyl or aromatic radical; and $R_2$ and $R_3$ independently represent hydrogen or an alkyl or an aromatic radical.

Figures 10 through 15 show various examples of structures of tracers in accordance with the present invention.

## DETAILED DESCRIPTION OF THE INVENTION

### Definitions

The term "ligand", as used herein, refers to a molecule, in particular a low molecular weight hapten, to which a binding protein, such as a receptor or an antibody, can be obtained or formed. Such haptens are protein-free compounds, generally of low molecular weight, which do not induce antibody formation when injected into an animal, but which are reactive to antibodies. Antibodies to haptens are generally raised by first conjugating the haptens to a protein and injecting the conjugate product into an animal. The resulting antibodies are isolated by conventional, well-known antibody isolation techniques.

The term "ligand-analog", as used herein, refers to a mono- or polyvalent radical, a substantial portion of which has the same spatial and polar organization as the ligand to define one or more determinant or epitopic sites capable of competing with the ligand for the binding sites of a receptor. A characteristic of such ligand-analog is that it possesses sufficient structural similarity to the ligand of interest so as to be recognized by the antibody for the ligand. For the most part, the ligand-analog will have the same or substantially the same structure and charge distribution (spatial and polar organization) as the ligand of interest for a significant portion of the molecular surface. Since frequently, the linking site for a hapten will be the same in preparing the antigen for production of antibodies as that used in the tracer for linking to the ligand, the same portion of the ligand analog which provides the template for the antibody will be exposed by the ligand analog in the tracer.

The term "fluorescein", as used herein, and in any Figure in which a fluorescein moiety occurs either as an individual compound or as a component of a larger compound, includes both the open and closed forms, is described in fra, if they exist for a particular molecule, except in the context of fluorescence. An open form is necessary for fluorescence to occur.

Ligands, Ligand-Analogs and Fluorescein

Ligands determinable by the method of the present invention vary over a wide molecular weight range. Although high molecular weight ligands may be determined, for best results, it is generally preferable to employ the methods of the present invention to determine ligands of relatively low molecular weight; i.e., within the range of from about 50 to about 4000

daltons. It is more preferred to determine ligands according to the invention which have a molecular weight within the range of from about 100 to about 2000.

Representative of ligands determinable by the methods of the present invention include steroids such as estriol estrone, estradiol, cortisol, testostrone, progesterone, deoxycholic acid, lithocholic acid and the ester and amide derivatives thereof; vitamins such as B-12 and folic acid; hormones such as thyroxine, triiodothyronine, histamine, serotonin; prostaglandins such as PGE, PGF, PGA; antiasthamatic drugs such as theophylline; antineoplastic drugs such as doxorubicin and methotrexate; antiarrhythmic drugs such as disopyramide, lidocaine, procainamide, propranolol, quinidine and N-acetyl-procainamide; anticonvulsant drugs such as phenobarbital, phenytoin, primidone, valproic acid, carbamazepine and ethosuximide; antibiotics such as penicillins, cephalosporins and vancomycin; antiarthritic drugs such as salicylates; antidepressant drugs, including tricyclics such as nortriptyline, amitriptyline, imipramine and desipramine; and immunosuppressive drugs such as cyclosporins and the like, as well as the metabolites thereof. Additional ligands determinable by the methods of the present invention include the so-called "drugs of abuse", such as morphine, heroin, hydromorphone, oxymorphone, metapon, codeine, hydrocodone, dihydrocodeine, dihydrohydroxycodeinone, pholcodine, dextromethorphan, phencyclidine, tetrahydrocannabinol, phenazocine and deonin, and their metabolites.

In general, the class of ligand-analogs represented in Figure 1 by Lig are derived from the corresponding ligand by removal of a hydrogen atom or a hydroxyl group at the site of binding to the group represented by R. Illustrative of ligands which upon

the removal of a hydrogen may form ligand-analogs represented by Lig include for example, procainamide, ethosuximide, phenobarbital and quinidine. Illustrative of ligands whose amino derivatives are useful as ligand-analogs are theophylline, valproic acid, phenobarbital, phenytoin, primidone, disopyramide, digoxin, chloramphenicol, salicylate, acetaminophen, carbamazepine, desipramine and nortriptyline. Illustrative of ligands where removal of a hydroxyl group produces useful ligand-analogs are digitoxigenin and digoxigenin. In addition, a ligand may be structurally modified by the addition, deletion, or substitution of one or more functional groups to form a ligand-analog which retains the necessary epitope sites for binding to an antibody. However, such modified ligand-analogs are normally bonded to the linking group R through the same site employed for non-modified ligand analogs. It is preferred that the linking group R be relatively short, normally in the range from zero to 4 atoms in length.

The present invention involves the use of fluorescein and derivatives of fluorescein. In particular, a necessary property of fluorescein and its derivatives for the usefulness of the tracer compounds is the fluorescence of fluorescein. Fluorescein exists in two tautomeric forms, illustrated in Figure 2, depending on the acid concentration (pH) of the environment. In the open (acid) form, there are a number of conjugated double bonds which make that form of fluorescein (and compounds containing a fluorescein moiety) capable of absorbing blue light and emitting green fluorescence after an excited state lifetime of about four nanoseconds. When the open and closed forms coexist, the relative concentration of molecules in the open and closed forms is easily altered by adjustment of

the pH level. Generally, the tracer compounds of the present invention exist in solution as biologically acceptable salts such as sodium, potassium, ammonium and the like, which allows the compounds to exist in the open, fluorescent form, when employed in the analytical methods of the present invention. The specific salt present depends on the buffer employed to adjust the pH level. for example, in the presence of a sodium phosphate buffer, the compounds of the present invention will generally exist in the open form, as a sodium salt.

The numbering of carbon atoms of the fluorescein molecule varies, depending upon whether the open or closed form of the molecule is considered. Accordingly, the literature concerning fluorescein and its compounds is not uniform as to carbon atom numbering. In the closed form, the para-carbon to the carbonyl of the lactone on the phenyl ring is numbered 6. In the open form, the para-carbon to the carboxylic acid group on the phenyl ring is numbered 5 (See Figure 2). In this disclosure the numbering of the closed form is adopted because the commercial raw materials used in the syntheses are most popularly numbered with that system. The carbon atom of fluorescein and its compounds which is opposite the carboxyl group is therefore numbered "6" for purposes of the present disclosure.

Fluorescence Polarization Immunoassay

In accordance with the method of the present invention, a sample containing the ligand to be determined is intermixed with a biologically acceptable salt of a tracer of formula (I) and an antibody specific for the ligand and tracer. The ligand present in the sample and the tracer compete for a limited number of antibody sites, resulting in the formation of ligand-antibody and tracer-antibody complexes. By

maintaining constant the concentration of tracer and antibody, the ratio of ligand-antibody complex to tracer-antibody complex that is formed is directly proportional to the amount of ligand present in the sample. Therefore, upon exciting the mixture with polarized light and measuring the polarization of the fluorescence emitted by a tracer and a tracer-antibody complex, one is able quantitatively to determine the amount of ligand in the sample.

A tracer in solution which is not complexed to an antibody is free to rotate in less than the time required for absorption, and re-emitted light is relatively randomly oriented so that the fluorescence polarization of a tracer not complexed to an antibody is low, approaching zero. Upon complexing with a specific antibody, the tracer-antibody complex thus formed assumes the rotation of the antibody molecule which is slower than that of the relatively small tracer molecule, thereby increasing the polarization observed. Therefore, when a ligand competes with the tracer for antibody sites, the observed polarization of fluorescence of the resulting mixture of the free tracer and tracer-antibody complex assumes a value intermediate between that of the tracer and that of the tracer-antibody complex. If a sample contains a high concentration of the ligand, the observed polarization value is closer to that of the free ligand, i.e., low. If the test sample contains a low concentration of the ligand, the polarization value is closer to that of the bound ligand, i.e., high. By sequentially exciting the reaction mixture of an immunoassay with vertically and then horizontally polarized light and analyzing only the vertically polarized component of the emitted light, the polarization of fluorescense in the reaction mixture can be accurately determined. The precise relationship

between polarization and concentration of the ligand to be determined is established by measuring the polarization values of calibrators with known concentrations. The concentration of the ligand can be interpolated from a standard curve prepared in this manner.

The pH at which the method of the present invention is practiced must be sufficient to allow the tracers of formula (I) to exist in their ionized state. The pH may range from about 3 to 12, more usually in the range of from about 5 to 10, most preferably from about 6 to 9. Various buffers can be used to achieve and maintain the pH during the assay procedure. Representative buffers include borate, acetate, phosphate, carbonate, tris, barbital and the like. The particular buffer employed is not critical to the present invention, but in an individual assay, a specific buffer may be preferred in view of the antibody employed and ligand to be determined. The cation portion of the buffer will generally determine the cation portion of the tracer salt in solution.

The methods of the present invention are practiced at moderate temperatures and preferably at a constant temperature. The temperature will normally range from about zero degrees to about 50 degrees C, more usually from about 15 degrees to about 40 degrees C.

The concentration of ligand which can be assayed in accordance with the invention will generally vary from about $10^{-2}$ to about $10^{-13}$ M, more usually from about $10^{-4}$ to about $10^{-10}$ M. High concentrations of ligand can be assayed upon dilution of the original sample.

In addition to the concentration range of ligand of interest, considerations such as whether the assay is qualitative, semiquantitative or quantitative,

the equipment employed, and the characteristics of the tracer and antibody will normally determine the concentration of the tracer and antibody which is used. While the concentration range of ligand in the sample will determine the range of concentration of the other reagents, i.e., tracer and antibody, normally to optimize the sensitivity of the assay, individual reagent concentrations will be determined empirically. Appropriate concentrations of the tracer and antibody are readily ascertained by one of ordinary skill in the art.

Although not forming part of the present invention, it is to be appreciated that fluorescence polarization immunoassays can be performed especially advantageously using reagents and assay procedures, in accordance with the invention, on a TDx (registered trademark) Fluorescence Polarization Analyzer, commercially available from Abbott Laboratories, Abbott Park, Illinois, from whom full details concerning its operation and features are available.

The Reagents

The tracers of the present invention can be represented by the general structural formula set forth in the Summary of the Invention, and illustrated in Figure 1.

a) The Structure of the Tracers

The preferred tracers of the present invention are characterized as derivatives of 5-carboxyfluorescein or (6-carboxyfluorescein or mixtures thereof, and are represented by the formulas shown in Figures 3 and 4. They are commonly prepared from the carboxyfluorescein starting materials shown and named in Figures 5 and 6, and may or may not be synthesized by way of the

piperazinocarboxyfluorescein intermediates having the structures shown and named in Figures 7 and 8.

A variety of chemical linkages can be employed in attaching the ligand analog to the 4-position nitrogen of the piperazine ring as depicted in Figure 9. The nitrogen can be bonded to a carbon atom which is attached only to hydrogen or other carbon atoms to form a tertiary amine as in Figure 9a. The 4-position nitrogen can be bonded to a carbon atom which forms part of a carbonyl group; in this case, if the remaining valence of this carbon is attached to another carbon atom, a tertiary carboxamide linkage is formed (Figure 9b); if the remaining valence is attached to an oxygen atom, a carbamate or urethane linkage is formed (Figure 9c); and if the remaining valence is attached to nitrogen, then the linkage is a urea (Figure 9d). If the 4-position nitrogen is attached to a carbon atom which forms part of a thiocarbonyl group, then a thioamide (Figure 9e) or thiourea (Figure 9f) link may be formed, depending on whether the remaining valence of the carbon is bonded to another carbon atom or to nitrogen. The 4-position nitrogen can be bonded to a carbon atom which is doubly bonded to nitrogen, forming an amidine (Figure 9g) or a guanidine (Figure 9h) depending upon whether the remaining valence of the carbon is attached to another carbon atom or to nitrogen. The 4-position nitrogen of the piperazine ring may also be bonded to sulfur in the +6 oxidation state, forming a sulfonamide (Figure 9j) or a sulfonylurea (Figure 9k), depending on whether the remaining valence of the sulfur is attached to carbon or to another nitrogen atom.

b) Synthesis of the Tracers

The chemical bonds in the tracers of the present invention between carboxyfluorescein and one of the piperazine ring nitrogens, between the linking group R (as depicted in Figure 1) and the other of the piperazine ring nitrogens and between the ligand analog Lig and the linking group R, can be established in any order which is synthetically convenient. It is frequently preferable to couple 5- or 6-carboxyfluorescein to piperazine to form the precursors depicted in Figures 7 and 8, activating the fluorescein carboxylic acid by reaction with a leaving group reagent such as N-hydroxysuccinide, 1-hydroxybenzotinazole, p-nitropiend and the like plus an activating reagent such as dicyclohexyl carbodiimide, diisopropyl carbodiimide and the like, and then allowing this activated form to react with piperazine. These piperazinocarboxyfluoresceins can be prepared in quantity and stored for use at a later time.

Coupling of the piperazine or one of the piperazinocarboxyfluoresceins to a ligand analog or an additional linking group to form a tertiary amine may be carried out in one of several ways. If the ligand analog or linking group bears an alkylating functionality with a leaving group such as bromide, iodide or sulfonate ester or the like, the coupling is carried out by alkylation, usually in the presence of some basic or alkaline reagent. If the ligand analog or linking group bears a carbonyl functionality such as a ketone or aldehyde, or the like, the coupling is carried out by condensation with the piperazine nitrogen followed by reduction with hydrogen and a catalyst or with a hydride reducing reagent such as borohydride or cyanoborohydride or the like; wherein the condensation and reduction steps can be carried out in a single reaction vessel.

A carboxamide bond to the piperazine nitrogen is normally established when the ligand analog or linking group bears a carboxylic acid. The coupling is carried out by activating the carboxylic acid as an acid halide such as an acid chloride or as an active ester in the manner described supra for the coupling of fluorescein carboxylic acid to piperazine, or by converting the acid to either a symmetrical or a mixed anhydride or the like and then allowing the activated form to react with piperazine or with one of the piperazinocarboxyfluoresceins.

A carbamate linkage between the piperazine and the ligand analog or linking group is normally established by joining the piperazine nitrogen to an alcoholic oxygen with the aid of phosgene or a phosgene equivalent reagent such as trichloromethyl chloroformate or carbonyl diimidazole or the like. In theory, it is possible to react either of the starting materials with the phosgene reagent, and then couple to the other. In practice it is generally preferred first to react the alcohol with the phosgene reagent and then to react the piperazine moiety with the resulting chloroformate or equivalent.

A urea linkage between the piperazine and the ligand analog is normally established by allowing an isocyanate or a carbamoyl chloride or equivalent derivative of the ligand analog to react with piperazine or a piperazinocarboxyfluorescein. The isocyanates can be prepared from primary amines by treatment with phosgene or a phosgene equivalent, such as trichloromethyl chloroformate or carbonyl diimidazole. Carbanoyl chlorides or their equivalents are prepared from secondary amines in the same manner. When the ligand analog contains additional functionality which would react with or be destroyed by phosgene or by the other

components of the phosgene reaction, it is generally preferred to establish the linkage by first reacting the phosgene or phosgene equivalent with mono-protected piperazine or with a protected piperazinocarboxyfluorescein, then reacting the carbamoyl chloride thus produced with the amino derivative of the ligand analog and finally removing the protecting group.

A thioamide linkage is normally established by heating an amide (preparation described supra) with phosphorus pentasulfide in an appropriate solvent. A thiourea linkage between the piperazine nitrogen and a nitrogen of the ligand analog is prepared in the same manner as for the urea linkage described above, except that thiophosgene is used instead of phosgene.

An amidine linkage is carried out by treating a nitrile derivative of the ligand analog with an anhydrous lower alcohol such as methanol or ethanol in the presence of hydrogen chloride. The resulting imino ester is then reacted with piperazine or a piperazinocarboxyfluorescein.

To establish a sulfonamide linkage, an activated sulfonic acid derivative of the ligand analog, such as a sulfonyl chloride, is reacted with piperazine or a piperazinocarboxyfluorescein. The sulfonyl chlorides are prepared from sulfonic acids by treatment with a chlorinating reagent such as phosphorus pentachloride, thionyl chloride, phosphoryl chloride and the like. Direct introduction of sulfonyl chloride groups into aromatic rings is accomplished by treatment with chlorosulfuric acid.

EXAMPLES

Examples 1 through 9 describe experiments which were performed in accordance with the concepts of the present invention, and are directed to the synthesis of tracers and precursors thereto. Examples 10 through 12 illustrate the suitability of tracers of the present invention, in immunoassays employing fluorescence polarization techniques. Such assays are conducted in accordance with the following general procedure:

1) A measured volume of standard or test serum is delivered into a test tube and diluted with buffer;

2) A known concentration of a tracer of the present invention optionally containing a surfactant is then added to each tube;

3) A known concentration of antisera is added to the tubes;

4) The reaction mixture is incubated at room temperature; and

5) The amount of tracer bound to antibody is measured by fluorescence polarization techniques as a measure of the amount of ligand in the sample.

<u>Example 1: 6-Piperazinocarboxyfluorescein</u>

A solution of 39 mg of 6-carboxyfluorescein, 17 mg of 1-hydroxybenzotriazole monohydrate and 62 mg of dicyclohexyl carbodiimide in 1.25 ml of dry pyridine was stirred at ice-water bath temperature for 0.5 hour and then at room temperature for 2.5 hours. This solution was filtered through glass wool into a solution of 86 mg of piperazine in 0.1 ml of dry pyridine. After stirring for 15 minutes, precipitation of the product was completed by addition of ethyl ether to give a bright red piperazinocarboxyfluorescein conjugate, having the structure shown in Figure 8.

Example 2: 5-Piperazinocarboxyfluorescein

This compound, having the structure shown in Figure 7, is made according to the method of Example 1.

Example 3: 6-Piperazinocarboxyfluorescein-phenobarbital Tracer

To a suspension of 25 mg of 5-phenyl-5-(2-hydroxyethyl)barbituric acid in 5 ml of a 10% solution of phosgene in benzene were added 2.5 ml of dry tetrahydrofuran. The mixture was stirred at room temperature for 2.5 hours, during which time the starting material gradually dissolved. The solvents and excess reagent were removed on a rotary evaporator, leaving the chloroformate as a colorless glass. A solution of 6-piperazinocarboxyfluorescein (prepared from 39 mg of 6-carboxyfluorescein as in Example 1) in 1.1 ml of dry dimethylformamide was added and the solution was stirred for 0.5 hours at room temperature. The product was precipitated by addition of about 20 ml of ether, redissolved in methanol, and chromatographed on a silicagel thick layer plate with chloroform-methanol to give a substantially pure product whose structure is shown in Figure 10.

Example 4: 6-Piperazinocarboxyfluorescein-ethosuximide Tracer

A solution of about 20 mg of 3-methyl-3-ethyl-1(3-hydroxy-1-propyl)succininimide in 1.0 ml of 10% phosgene-benzene was allowed to stand for 2.5 hours at ambient temperature, and then the solvent and excess reagent were removed. A solution of 6-piperazinocarboxyfluorescein in 0.1 ml of DMF was added and the solution was again allowed to stand for 2 hours at ambient temperature. The product was chromatographed on a silicagel thin layer plate developed with benzene-ethyl acetate-acetone to give the pure tracer whose structure is shown in Figure 11.

Example 5: 3-Deoxy-3-(N-piperazino)digitoxigenin

A mixture of 186.5 mg of digitoxigenin-3-one, 557 mg of piperazine dihydrochloride and 36 mg of sodium cyanoborohydride in 10 ml of anhydrous methanol was stirred at ambient temperature under nitrogen atmosphere for 14 days. The reaction was quenched by addition of a few drops of 3 M hydrochloric acid and the solvents were removed. The residue was taken up in 0.5 M hydrochloric acid and filtered. The pH of the remaining solution was adjusted to 8.5 and it was extracted exhaustively with dichloromethane. Drying and removal of solvent left 80 mg of the 3-piperazino derivative.

Example 6:
3-Deoxy-3-(5-piperazinocarboxyfluorescein)digitoxigenin

A solution of 19.7 mg of 5-carboxyfluorescein, 20.6 mg of dicyclohexylcarbodiimide and 7 mg of N-hydroxysuccinimide in 0.25 ml of pyridine was allowed to stand at ambient temperature for 5 hours. The precipitate which appeared was removed and the solution was added to 22 mg of 3-deoxy-3-(N-piperazino) digitoxigenin. The reaction was allowed to proceed at ambient temperature overnight, and pure product, whose structure is represented by Figure 12, was isolated by chromatography on thick layer silicagel plates developed with chloroform-methanol.

Example 7: 3-Deoxy-3-(6-piperazinocarboxyfluoresceinyl)-digitoxigenin

This compound, whose structure is represented in Figure 13, was prepared according to the method of Example 6.

Example 8: 6-Piperazinocarboxyfluorescein-Oxazepam Carbamate Tracer

Oxazepam was converted to the corresponding chloroformate by dissolving 15 mg of oxazepam in 1.0 ml of dry tetrahydrofuran, adding 0.4 ml of 10% phosgene in benzene and 0.008 ml of pyridine and stirring overnight at ambient temperature. Solvents and excess reagent were removed on a rotary evaporator and the residue was taken up in 0.2 ml of dimethylformamide. A solution of 6-piperazinocarboxyfluorescein (freshly prepared from 20 mg of 6-carboxyfluorescein according to the method of Example 1) in 0.2 ml of dimethylformamide was added, followed by 0.1 ml of pyridine, and the mixture was stirred at ambient temperature for 2.5 days. The crude material was purified by chromatography over a silicagel thick layer plate developed with chloroform methanol to give a substantially pure tracer, having the structure represented in Figure 14.

Example 9: 6-Piperazinocarboxyfluorescein-Digitoxigenin Carbamate Tracer

Digitoxigenin was converted to the corresponding chloroformate by dissolving 9.4 mg of digitoxigenin in 0.2 ml of dry tetrahydrofuran and allowing it to react with about 0.12 ml of 10% phosgene in benzene for 3 hours at room temperature. Solvents and excess reagent were removed on a rotary evaporator, and the residue was dissolved in 0.1 ml of dry dimethylformamide and mixed with 6-piperazinocarboxyfluorescein (freshly prepared from 14.8 mg of 6-carboxyfluorescein according to the method of Example 1) in another 0.15 ml of dimethylformamide. The solution was allowed to stand at ambient temperature overnight and the crude product was purified by chromatography on a thick layer silicagel plate

developed with hexane-acetone-acetic acid and then with chloroform methanol to give the tracer having the structure shown in Figure 15.

Example 10: Phenobarbital Assay

A) Materials Required

1) BGG Buffer containing 0.1 M sodium phosphate, pH 7.5; 0.01% (w/v) bovine gamma globulin and 0.1% (w/v) sodium azide.

2) Tracer, consisting of 6-piperazimocarboxyfluoresceins-phenobarbital carbamate conjugate at a concentration of approximately 120nM in 0.1 M Tris buffer pH 7.5; 0.1% (w/v) sodium azide; 0.01% (w/V) bovine gamma globulin; 25% (v/v) dimethylformamide and oleic acid at 100 microgram/mL.

3) Antiserum, consisting of antiserum raised against phenobarbital-bovine serum albumin conjugate, diluted appropriately in BGG buffer containing 2% ethylene glycol.

4) Pretreatment solution, containing 0.1 M Tris, pH 7.5; 0.1% (w/v) sodium azide; 0.01% (w/v) bovine gamma globulin and 0.1% (w/v) sodium dodecylsulfate.

5) Calibrators, consisting of appropriate concentrations of phenobarbital dissolved in normal human serum.

B) Assay Protocol

1) A small aliquot (0.6 microliters) of the calibrator or unknown sample to be quantitated is mixed with 25 microliters of pretreatment solutions and diluted to 1.0 ml with BGG buffer. The polarized fluorescence background is measured at this point.

2) To the assay solution from step 1 is added a second 0.6 microliter aliquot of the sample, together with 25 microliters of tracer solution, 25 microliters of antiserums solution and buffer to a final volume of 2.0 mL. The final polarized fluorescence is then read.

3) The fluorescence polarization due to tracer binding is obtained by subtracting the polarize4d fluorescence of the background from that of the final assay mixture.

4) The fluorescence polarization value for an unknown sample is compared to a standard curve constructed by the use of calibrators, and the unknown concentration is determined by interpolation

C) Results

Typical results for a series of human serum calibrators containing phenobarbital at concentrations between zero and 50 micrograms per milliliter (ug/ml) are presented below. Each concentration was assayed in duplicate on a TDx Analyzer, and the polarization values were averaged.

| Concentration of phenobarbital (ug/mL) | Polarization reading |
|---|---|
| 0 | 0.219 |
| 5 | 0.194 |
| 10 | 0.169 |
| 20 | 0.138 |
| 40 | 0.108 |
| 80 | 0.084 |

The average polarization is seen to decrease in a regular manner as phenobarbital concentration increases, allowing the construction of a smooth standard curve and demonstrating the value of this tracer for quantitation of phenobarbital in serum samples.

Example 11: Ethosuximide Assay

A) Materials Required

1) BGG Buffer (see Example 10)

2) Tracer solution, consisting of 6-piperazinocarboxyfluorescein-ethosuximide conjugate at a concentration of approximately 82 nanomolars in BGG buffer.

3) Antiserum solution, consisting of antiserum raised against ethosuximide-protein conjugate, diluted appropriately with BGG buffer.

4) Pretreatment solution, containing 5% (w/v) 5-sulfosalicylic acid in 0.1 M Tris (pH 5.5) buffer containing 0.1% sodium azide.

5) Calibrators, consisting of appropriate concentrations of ethosuximide dissolved in normal human serum.

B) Assay Protocol

The assay is performed in substantially the same manner as for phenobarbital (Example 10) except that 5 microliter aliquots of sample are used in steps 1 and 2.

C) Results

Typical results for a series of human serum calibrators containing ethosuximide at concentrations between zero and 150 micrograms per milliliter are presented below. Each concentration was assayed on a TDx Analyzer as described in Example 10, in duplicate, and the polarization values were averaged.

| Concentration of ethosuximide ug/mL | Average polarization |
|---|---|
| 0 | 0.196 |
| 12.5 | 0.192 |
| 25 | 0.188 |
| 50 | 0.182 |
| 100 | 0.178 |
| 150 | 0.175 |

The average polarization is seen to decrease in a regular manner as ethosuximide concentration increases, allowing the construction of a smooth standard curve and demonstrating the value of this tracer in quantitation of ethosuximide in serum samples.

Example 12: Digitoxin Assay

A) Materials Required

1) BGG Buffer (see Example 10)

2) Tracer, consisting of

fluorescein-piperazine-digitoxigenin conjugate diluted to an appropriate concentration in buffer with appropriate additions.

3) Antiserum, consisting of antiserum raised against a digitoxin-protein conjugate and diluted appropriately in BGG buffer containing 2% ethylene glycol.

4) Extractant solution, consisting of a 5% (w/v) solution of trichloroacetic acid in water.

5) Pretreatment solution, consisting of 20% (w/v) salicylic acid in BGG buffer.

6) Calibrators, consisting of 0, 5, 10, 20, 30, and 50 nanogram/mL solutions of digitoxin in pooled normal human serum.

7) A centrifuge capable of attaining a force of 9,500 x g together with appropriate centrifuge tubes.

B) Assay Protocol

1) A 200 microliter aliquot of each calibrator or unknown sample is pipetted into a centrifuge tube, treated with 200 microliters of extractant solution, mixed thoroughly and centrifuged for 3 minutes.

2) A 175 microliter aliquot of this solution is prediluted with 25 microliters each of antiserum and pretreatment solutions.

3) A volume of prediluted sample equivalent to 60 microliters of the supernatant from the centrifugation of step 1 is made up to a volume of 1.0 mL with BGG buffer and the polarized fluorescence background is measured.

4) To the assay solution from step 3 is added a second, equal volume of prediluted sample, together with 25 microliters of tracer solution, and buffer to a final volume of 2.0 mL. The final polarized fluorescence reading is then taken.

5) The fluorescence polarization value for an unknown sample is compared to a standard curve constructed employing the calibrators, and the unknown concentration is determined by interpolation.

C) Results

Typical results for a series of human serum calibrators containing digitoxin at concentrations between zero and 50 nanogram/mL are presented below. Each concentration was assayed in duplicate using a TDx Analyzer as previously described and the polarization values were averaged.

| | Polarizations | |
|---|---|---|
| Concentration of digitoxin ng/ml | Tracer of Figure 12 | Tracer of Figure 13 |
| 0 | 0.134 | 0.127 |
| 5 | 0.124 | 0.117 |
| 10 | 0.116 | 0.112 |
| 20 | 0.100 | 0.098 |
| 30 | 0.085 | 0.083 |
| 50 | 0.063 | 0.065 |

As digitoxin concentration increases the average polarization can be seen to decrease in a regular manner, allowing the construction of a smooth standard curve and demonstrating the value of these tracers in quantitations of digitoxin in serum samples.

It is apparent that various modifications and variations can be made by one skilled in the art from the specific disclosure of the invention herein contained, without departing from the spirit and scope of the invention, as defined solely in the following claims.

CLAIMS:

1. A method for determining the presence or concentration of a ligand in a liquid sample comprising intermixing with the sample a tracer of the formula:

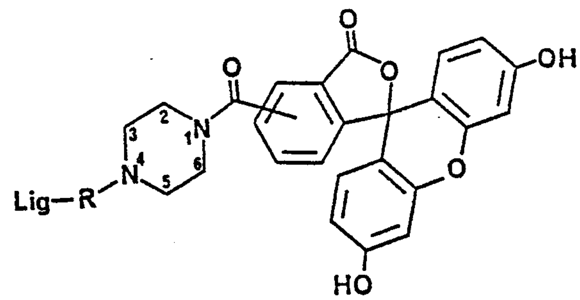

wherein Lig is a ligand analog which has at least one common epitope with the ligand so as to be specifically recognizable by a common antibody or receptor binding site; and

R is a linking group consisting of from zero to 12 carbon, nitrogen, sulfur or oxygen atoms, together with sufficient hydrogen atoms to satisfy valences of the above-named atoms which are not otherwise satisfied

with a solution of an antibody capable of specifically recognizing the ligand and the tracer; and determining the amount of the tracer which becomes bound to the antibody by fluorescence polarization as a measure of the amount of ligand in the sample.

2. A method according to Claim 1 wherein the ligand has a molecular weight within the range of 50 to 4000.

3. A method according to Claim 1 wherein the ligand is a drug or a hormone, or a metabolite thereof.

4. A method according to Claim 3 wherein said drug is an anticonvulsant, a cardiac glycoside, a tranquilizer, an antidepressant, an antiasthmatic, an antihypertensive, an antineoplastic, an antiarrhythmic, a stimulant, a narcotic, a psychotropic or an anabolic.

5. A method according to Claim 4 wherein said drug is phenobarbital.

6. A method according to Claim 4 wherein said drug is ethosuximide.

7. A method according to Claim 4 wherein said drug is digitoxin.

8. A method according to Claim 4 wherein said drug is oxazepam.

9. A compound useful in a fluorescence polarization assay for determining the presence or amount of a ligand in a liquid sample, which compound has the formula:

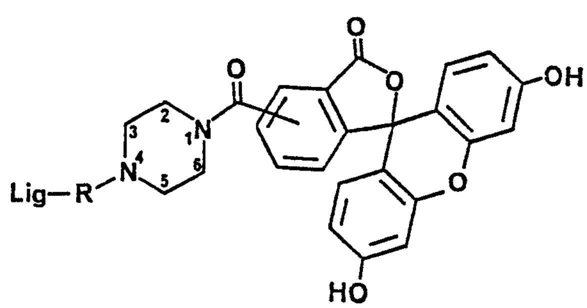

wherein Lig is a ligand analog having at least one common epitope with the ligand so as to be specifically recognizable by a common antibody or receptor binding site; and

R is a linking group consisting of from zero to 12 carbon, nitrogen, sulfur and oxygen atoms, together with sufficient hydrogen atoms to satisfy valences of the above-named atoms which are not otherwise satisfied, and biologically acceptable salts thereof.

10. A compound according to Claim 9 wherein Lig is an analog of phenobarbital.

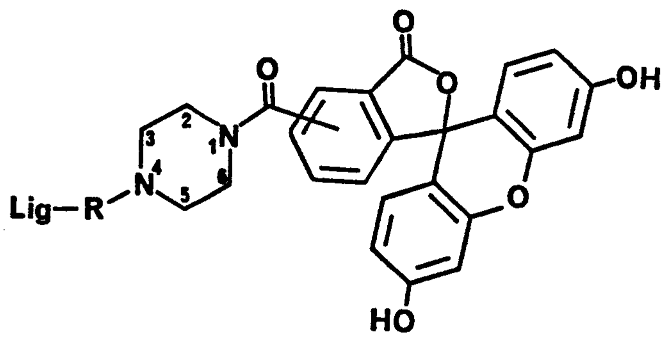

Fig. 1

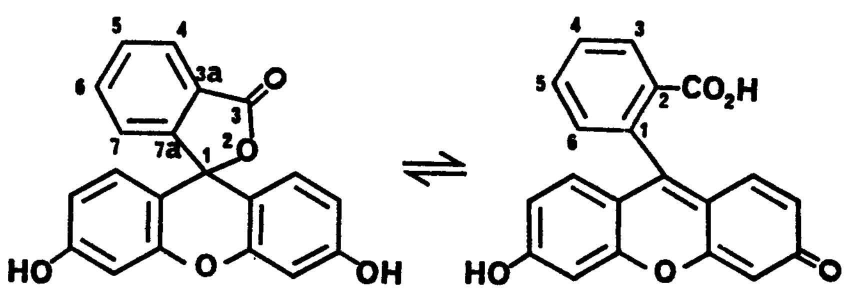

Lactone

Acid

Fig. 2

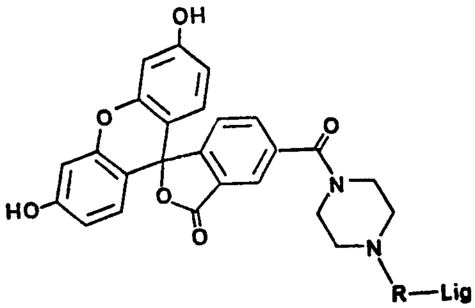

Fig. 3

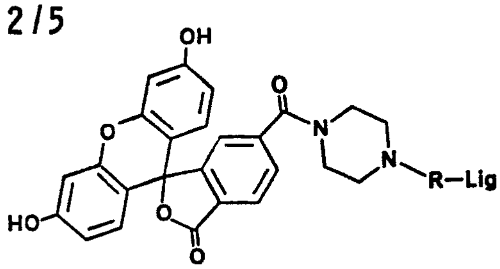

Fig. 4

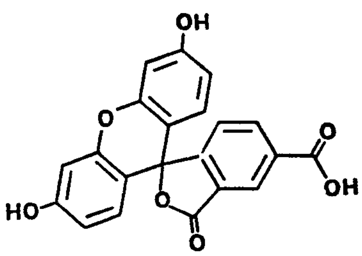

5-Carboxyfluorescein
Fig. 5

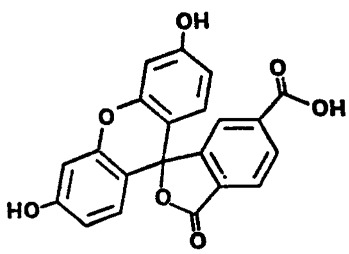

6-Carboxyfluorescein
Fig. 6

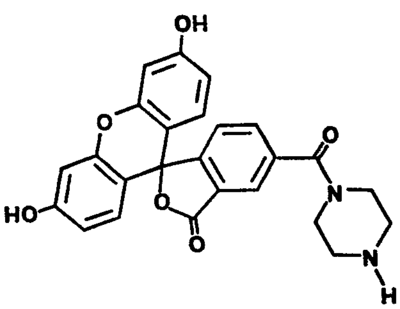

5-Piperazinocarboxyfluorescein
Fig. 7

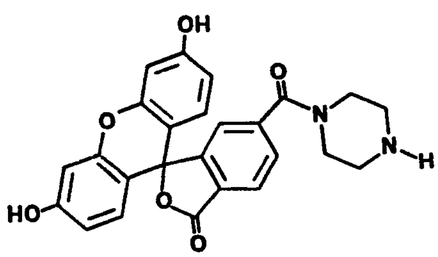

6-Piperazinocarboxyfluorescein
Fig. 8

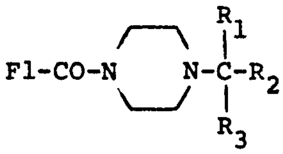

Fig. 9a

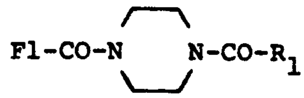

Fig. 9b

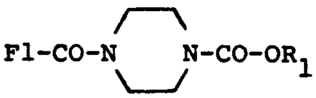

Fig. 9c

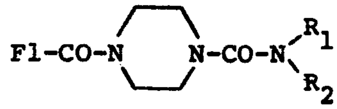

Fig. 9d

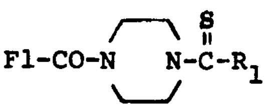

Fig. 9e

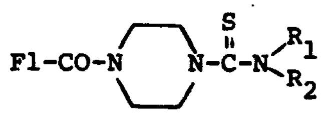

Fig. 9f

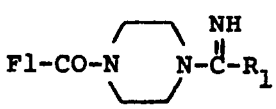

Fig. 9g

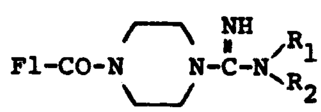

Fig. 9h

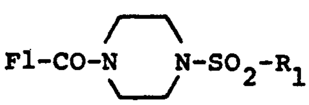

Fig. 9j

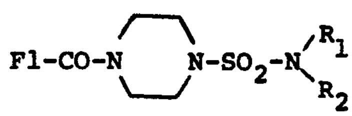

Fig. 9k

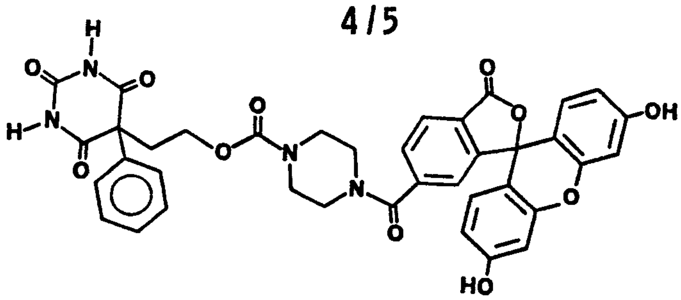

Fig. 10

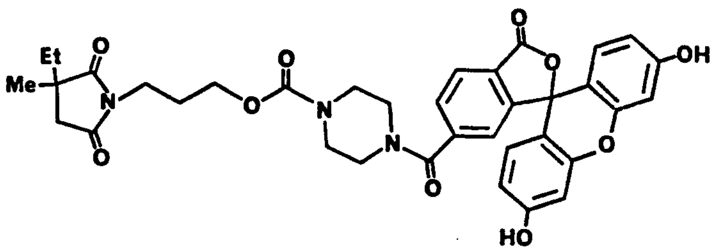

Fig. 11

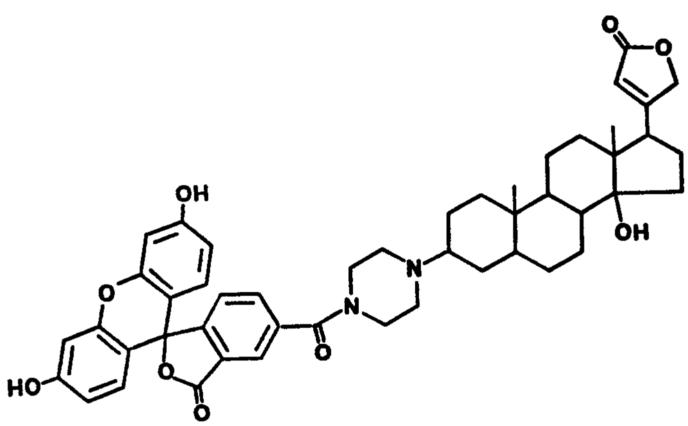

Fig. 12

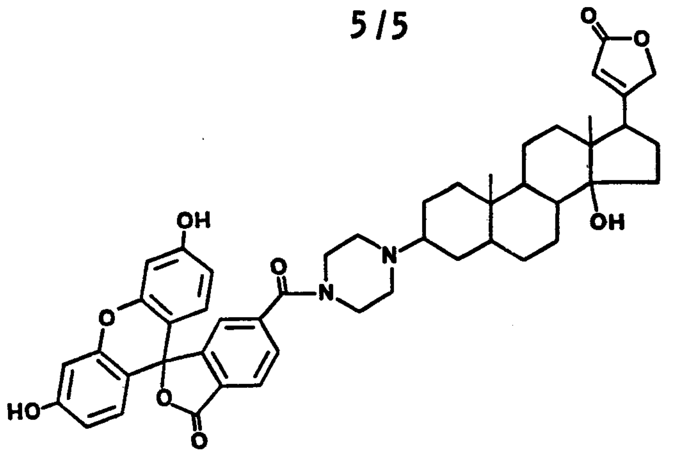

Fig. 13

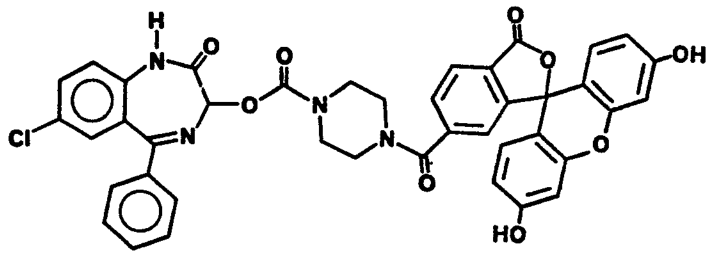

Fig. 14

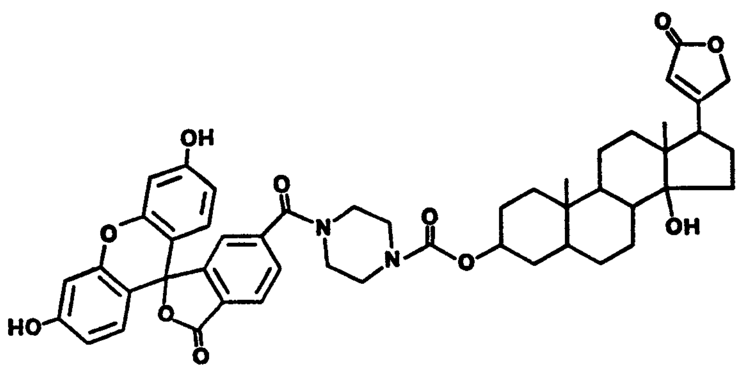

Fig. 15